# EUROPEAN PATENT APPLICATION

(11) **EP 1 136 472 A1**
(43) Date of publication of application: **26.09.2001**
(21) Application number: 98966999.9
(22) Date of filing: 04.12.1998
(51) Int. Cl.: C07C 311/44, C07D 249/08, C07D 251/04, C07D 285/08

(54) **ARYL- AND HETEROARYLAMIDES OF CARBOALKOXYSULFANILIC ACIDS**

(71) Applicant: Ashkinazi, Rimma Iliinichna, St. Petersburg, 191025 (RU); Krutikov, Viktor Iosifovich, St. Petersburg, 193015 (RU)
(72) Inventor: Ashkinazi, Rimma Iliinichna, St. Petersburg, 191025 (RU); Krutikov, Viktor Iosifovich, St. Petersburg, 193015 (RU)
(74) Representative: Zellentin, Rüdiger, Dr.
(86) International application number: RU9800385
(87) International publication number: WO0034234

(57) **Abstract**

The invention relates to medicine, and more specifically to pharmacology, and in particular to synthetic biologically active compounds of heterocyclic series, and it is designated mainly for use in medicine practice.

The objective of the invention is obtaining new chemical substances possessing wide range of biological activities, including antiviral activity (regarding *Herpes Simplex virus*), immune stimulating activity (for the expense of inducing production of endogenic interferons in a body), and antichlamydial, hepatoprotective, antiaggregational, and antimicrobial activities. In other words, the objective of the invention is to synthesize new biologically active compounds that outperform known analogs in some characteristics and range of action. The proposed objective is achieved by synthesis of aryl- and heterylamides of carboalkoxysulfanilic acids of the general formula (1) 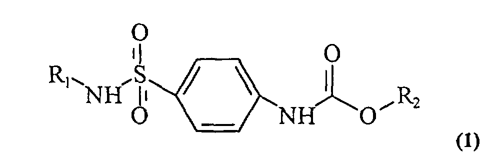 where: R₁ is taken from the group of aryls or heteryls; R₂ is taken from the group of alkyls.

General method of producing the claimed compounds and results of identification, a list of 42 compounds synthesized and tested, and experimental data on comparative testing their biological properties and toxicity are presented.

1 independ. and 42 depend. items of the formula, 9 tables, 10 ref.

## Description

### FIELD OF THE INVENTION

The invention relates to medicine, and more specifically to pharmacology, and in particular to synthetic biologically active compounds of heterocyclic series, possessing antiviral, immune-stimulating (interferon-inducing), antichlamydial, antiaggregational, hepatoprotective, and antimicrobial activities.

Compounds are mainly intended for use in medicine practice for treating viral infections; the infections caused by chlamydia; the diseases followed by immune-deficiency, in particular, malignant neoplasms; and also tuberculosis.

Besides that, the given compounds can be used in veterinary for the same purposes.

### TECHNICAL LEVEL

It is known that microbial and viral diseases are one of the most significant problems of modern medicine. Most of them hardly respond to treatment. It is connected with lack of efficiency of current drugs as well as high rate of variability of microorganisms that leads to origination of resistant forms [1, 2].

Viral diseases often take a course on the background of lowering activity of the immune system, and they are followed by secondary infections; the same is also true for oncologic diseases. Therefore the problem of the development of effective antiviral or antitumoral drugs is closely related with the searching for remedies intended for treating the immune-deficient states of various origination. As is known, stimulators of immune system are used in treating a number of oncologic diseases.

Existing antiviral drugs may be conventionally divided into two groups according to types of mechanisms of their action. An action of the drugs of the first group involves suppression of virus reproduction in a body [1]. Antiviral drugs of the second group produce their effect through stimulating the body's immune protection and increase of producing endogenic interferons [3] to a greater extent rather than affecting the viruses in themselves. Interferons and their inducers are also used for treatment of a various tumoral diseases [4].

The given invention falls into the second group.

Chemical analogs of the claimed compounds are aryl-heteryl-sulfamides: Ethazol, Diacarb,

Sulfadimidine 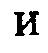 Butazolamid. Let's consider them more detailed.

Ethazol is 2-(4-aminobenzolsulfamido)-5-ethyl-1,3,4-thiadiazole.

Ethazol is known for its antibacterial activity in respect to streptococci, pneumococci, meningococcil, gonococci, collibacilli, and pathogenic anaerobic microorganisms. It is described in [5].

Diacarb is 2-acethylamino-1,3,4-thiadiazole-5-sulfamide, and it has the following structural chemical formula:

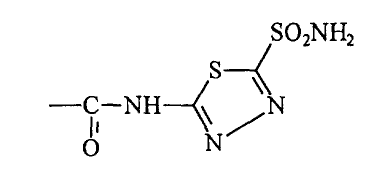

Diacarb is used as diuretic remedy, and also for treatment of glaucoma. It is described in [5] too.

Sulfadimidine is 2-(4-aminobenzolesulfamido)-4,6-dimethylpyrimidine. It has the following structural chemical formula:

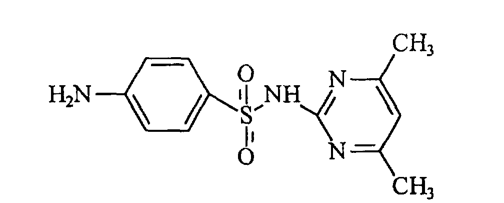

Sulfadimidine is used for treatment of pneumococcal, streptococcal, meningococcal infections, sepsis, gonorrhea, and also infections caused by collibacillus, and other microorganisms. It is described in [5] too.

The closest, by its chemical nature and designation, analog to the claimed compounds is Butazolamid, which is 2-butyrylamino-1,3,4-thiadiazole-5-sulfamide. It has the following structural chemical formula:

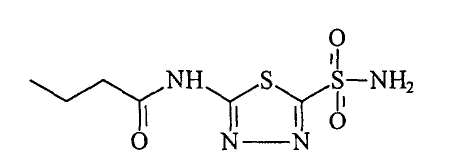

Butazolamid is used only as antibacterial agent. It is described in detail in [6].
Unfortunately, range of its biological activity is comparatively narrow; besides that, bacteria resistant to Butazolamid have already appeared.

### OBJECTIVE OF THE INVENTION

An objective of the invention is obtaining new chemical compounds possessing a wide range of biological activity, including antiviral activity (with respect to *Herpes Simplex virus*), immune-stimulating activity (at the expense of induction of producing endogenic interferons in a body), antichlamydial, hepatoprotective, antiaggregational, and antimicrobial activities. In other words, the objective of the invention is to provide chemical synthesis of biologically active compounds that outperform the prototype in their antiviral, immune-stimulating, antimicrobial activities, and also in the range of action.

### SUBJECT OF THE INVENTION

The proposed objective is achieved by synthesis of new chemical compounds - aryl- and heterylamides of carboalkoxysulfanilic acids of the general formula (1) 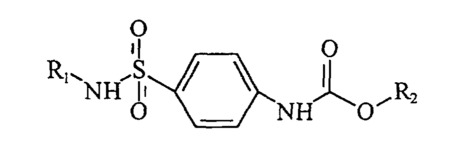 where:
- R₁: is taken from the group of aryls or heteryls;
- R₂: is taken from the group of alkyls.

A list of aryl- and heterylamides of carboalkoxysulfanilic acids, synthesized and tested by us, is presented in Table 1.

**Table 1**

| *. The claimed compounds: aryl- and heterylamides of carboalkoxysulfanilic acids* | | | |
|---|---|---|---|
| Number and name of compound | R₁ | R₂ | M.P., °C |
| I - Carbomethoxysulfanylic acid (4-bromophenyl)amide | 4-BrPh | CH₃ | >260 degrad. |
| II - Carboethoxysulfanylic acid (4-bromophenyl)amide | 4-BrPh | C₂H₅ | 232 |
| III - Carbobutoxysulfanylic acid (4-bromophenyl)amide | 4-BrPh | C₄H₉ | 185 |
| IV - Carbopentoxysulfanylic acid (4-bromophenyl)amide | 4-BrPh | C₅H₁₁ | 116 |
| V - Carbohexoxysulfanylic acid (4-bromophenyl)amide | 4-BrPh | C₆H₁₃ | 114 |
| VI - Carboheptoxysulfanylic acid (4-bromophenyl)amide | 4-BrPh | C₇H₁₅ | 143 |
| VII - Carbobutoxysulfanylic acid (4-nitrophenyl)amide | 4-NO₂Ph | C₄H₉ | 161 |
| VIII - Carbobutoxysulfanylic acid (2-toluyl)amide | 2-CH₃Ph | C₄H₉ | 113 |
| IX - Carbobutoxysulfanylic acid (2-chlorophenyl)amide | 2-ClPh | C₄H₉ | 112 |
| X - Carbobutoxysulfanylic acid (2,6-dichlorophenyl)amide | 2,6-Cl₂Ph | C₄H₉ | 121 |
| XI - Carbobutoxysulfanylic acid (2-iodophenyl)amide | 2-IPh | C₄H₉ | 81 |
| XII - Carbobutoxysulfanylic acid (3-bromophenyl)amide | 3-BrPh | C₄H₉ | 130 |
| XIII - Carbobutoxysulfanylic acid phenylamide | Ph | C₄H₉ | 140 |
| XIV - Carbobutoxysulfanylic acid (2,5-dimethyl)amide | 2,5-(CH₃)₂Ph | C₄H₉ | 117 |
| XV - Carbobutoxysulfanylic acid (4-fluorophenyl)amide | 4-FPh | C₄H₉ | 134 |
| XVI - Carbopentoxysulfanylic acid (1,2,4-triazole-4-yl)amide | 1,2,4-Triazole-4-yl | C₅H₁₁ | 144 |
| XVII - Carbopentoxysulfanylic acid (2-toluyl)amide | 2-CH₃Ph | C₅H₁₁ | 109 |
| XVIII - Carbopentoxysulfanylic acid (2-chlorophenyl)amide | 2-ClPh | C₅H₁₁ | 109 |
| XIX - Carbopentoxysulfanylic acid (2,6-dichlorophenyl)amide | 2,6-Cl₂Ph | C₅H₁₁ | 106 |
| XX - Carbopentoxysulfanylic acid (2-iodophenyl)amide | 2-IPh | C₅H₁₁ | 108 |
| XXI - Carbopentoxysulfanylic acid (3-bromophenyl)amide | 3-BrPh | C₅H₁₁ | 114 |
| XXII - Carbopentoxysulfanylic acid phenylamide | Ph | C₅H₁₁ | 101 |
| XXIII - Carbopentoxysulfanylic acid (2,5-dimethylphenyl)amide | 2,5-(CH₃)₂Ph | C₅H₁₁ | 108 |
| XXIV - Carbopentoxysulfanylic acid (4-fluorophenyl)amide | 4-FPh | C₅H₁₁ | 118 |
| XXV - Carbomethoxysulfanylic acid (2-toluyl)amide | 2-CH₃Ph | CH₃ | 154 |
| XXVI - Carboethoxysulfanylic acid (1,2,4-triazole-4-yl)amide | 1,2,4-Triazole-4-yl | C₂H₅ | 189 degrad. |
| XXVII - Carboheptoxysulfanylic acid (2-chlorophenyl)amide | 2-ClPh | C₇H₁₅ | 102 |
| XXVIII - Carbohexoxysulfanylic acid (2-chlorophenyl)amide | 2-ClPh | C₆H₁₃ | 104 |
| XXIX - Carbohexoxysulfanylic acid (2-toluyl)amide | 2-CH₃Ph | C₆H₁₃ | 112 |
| XXX - Carbohexoxysulfanylic acid (2,5-dimethylphenyl)amide | 2,5-(CH₃)₂Ph | C₆H₁₃ | 109 |
| XXXI - Carbohexoxysulfanylic acid phenulamide | Ph | C₆H₁₃ | 99 |
| XXXII - Carbobutoxysulfanylic acid (1,2,4-thiadiazole-2-methyl-5-yl)amide | 1,2,4-Thiadiazole-2-methyl-5-yl | C₄H₉ | 242 degrad. |
| XXXIII - Carbomethoxysulfanylic acid (1,2,4-thiadiazole-2-butyl-5-yl)amide | 1,2,4-Thiadiazole-2-butyl-5-yl | CH₃ | 214 degrad. |
| XXXIV - Carboheptoxysulfanylic acid (1,2,4-thiadiazole-2-hexyl-5-yl)amide | 1,2,4-Thiadiazole-2-hexyl-5-yl | C₇H₁₅ | 187 degrad. |
| XXXV - Carboisobutoxysulfanylic acid (1,2,4-thiadiazole-2-pentyl-5-yl)amide | 1,2,4-Thiadiazole-2-pentyl-5-yl | isoC₄H₉ | 169 degrad. |
| XXXVI - Carbomethoxysulfanylic acid (1,2,4-thiadiazole-2-methyl-5-yl)amide | 1,2,4-Thiadiazole-2-methyl-5-yl | CH₃ | 250 degrad. |
| XXXVII - Carbohexoxysulfanylic acid (1,2,4-thiadiazole-2-butyl-5-yl)amide | 1,2,4-Thiadiazole-2-butyl-5-yl | C₆H₁₃ | 143 degrad. |
| XXXVIII - Carbobutoxysulfanylic acid (1,2,4-triazole-4-yl)amide | 1,2,4-Triazole--4-yl | C₄H₉ | 190 degrad. |
| XXXIX - Carbo(2,2,2-trifluorethoxy)-sulfanylic acid (1,2,4-thiadiazole-2-pentyl-5-yl)amide | 1,2,4-Thiadiazole-2-pentyl-5-yl | CH₂CF₃ | 192 degrad. |
| XL - Carbo(2,2,2-trifluorethoxy)-sulfanylic acid (1,2,4-thiadiazole-2-butyl-5-yl)amide | 1,2,4-Thiadiazole-2-butyl-5-yl | CH₂CF₃ | 189 degrad. |
| XLI - Carbopentoxysulfanylic acid (1,5-dimethyl-2-phenylpyrazolone-4-yl)-amide | 1,5-Dimethyl-2-phenylpyrazolo ne-4-yl | C₅H₁₁ | 134 degrad. |
| XLII - Carbopentoxysulfanylic acid (1,3,5-triazine-2,4-diamino-6-yl)amid | 1,3,5-Triazine-2,4-diamino-6-yl | C₅H₁₁ | >250 degrad. |

The compounds claimed are new, because they are not known from available information sources.

The presence of wide range of an effective biological activities of the claimed compounds does not obviously stem from preceded level of technique, i.e. the presence of the indicated activities is not obvious for a specialist.

### DISCLOSURE OF THE INVENTION

The subject matter of the present invention is explained below by:
General method of producing the claimed compounds
Example of synthesizing carbomethoxysulfanilic acid (4-bromophenyl)amide (1)
Data on PMR spectroscopic study of compounds I-XLII (with Table 2)
Data on experiments for determining the biological activity of the claimed compounds as compared with widely used remedies of the same designation; that experiments are:
**Experiment 1.** Determination of antiviral effect of the claimed compounds, illustrated by the example of *Herpes Simplex virus* (with Table 3).
**Experiment 2.** Determination of interferon-inducing activity of the claimed compounds (with Table 4).
**Experiment 3.** Determination of the action of the claimed compounds against chlamydia, illustrated by the example of *Chlamydia trachomatis* (with Table 5).
**Experiment 4.** Determination of hepatoprotective activity of the claimed compounds (with Table 6).
**Experiment 5.** Determination of antiaggregational properties of the claimed compounds (with Table 7).
**Experiment 6.** Determination of antimicrobial action of the claimed compounds (with Table 8).
**Experiment 7.** Determination of the maximal tolerant dose of the claimed compounds (with Table 9).

### General method of producing compounds I-XLII

The method of obtaining the target substituted amides of carboalkoxysulfanilic acids is based on interaction between phenylisocyanate, carbamates, chlorsulfonic acid, and substituted aromatic and heterocyclic amines, conducting by stages.

### I stage

Phenylisocyanate is taken, then the corresponded alcohol (R₂OH), taken in molar ratio 1:1, is added drop by drop while mixing. The reaction mixture solidifies into colorless crystals through 1 to 10 hours (depending on reactivity of the alcohol). Output of the target product is quantitative. The purity of the obtained phenylcarbamic acid ester is sufficient to use it further.

### II stage

Carbamate is slowly added to chlorsulfonic acid heated to 28-30°C, while stirring, and keeping the temperature of the reaction mixture not higher than 50-55°C; the reaction mixture is kept at this temperature for 2 hours. Obtained sulfonated material is, while stirring, poured onto ice, keeping the temperature of the mixture about 20°C. Finely divided precipitation, that is usually formed as colorless, is filtered and washed with ice water, until a neutral reaction of filtrate is achieved. The residue is dried in air, and then in a vacuum-exsiccator over phosphorus pentoxide.

### III stage

Carboalkoxysulfanyl chloride obtained in previous stage is added in batches to a mixture of substituted aryl- or heterylamine (where R₁ is chosen depending on the target product) and pyridine at the temperature 82-88 C. During the introducing of the last batches, pH of the medium is checked. If pH is about 1-2, pyridine is added until alkaline reaction is produced. Then, hot water is added to the reaction mixture, the reaction mixture is acidified with hydrochloric acid until pH 3.0-3.5, and then the mixture is cooled to 20°C. Obtained residue is filtered, washed with water, and dried. Product output is about 30%.

### Example of synthesizing carbomethoxysulfanilic acid (4-bromophenyl)amide (1)

I stage. 16 g of methanol is added drop by drop to 59.5 g of phenylisocyanate, placed into a glass, while stirring. Warm-up of the reaction mixture is observed. The mixture solidifies through 1 hour in colorless crystals. Output of phenylcarbamic acid methyl ester is quantitative.

II stage. 76 g of methyl ester of phenylcarbamic acid is slowly added to 241 g of chlorsulfonic acid heated to 30°C, while stirring, and keeping the temperature of the reaction mixture not higher than 35°C. Then the mixture is slowly heated to 50°C and kept it at 50-55°C for 2 hours.

Obtained sulfonated material is, while stirring, poured out onto ice, keeping the temperature of the mixture not above 20°C. Finely divided colorless precipitation that is formed is filtered and washed with ice water, until pH of filtrate is come to 7. The residue is dried in air conditions, and then in a vacuum-exsiccator over phosphorus pentoxide. Output of carbomethoxysulfanylic chloride is 75 g (60% of theoretical one).

III stage. 35g of carbomethoxysulfanilic chloride is added in batches to a mixture of 17.2 g of 4-bromaniline and 15.8 g of pyridine at the temperature 85°C. To the end of adding the chloride, pH of the medium was 9.
Then, hot water is added to the reaction mixture, the reaction mixture is acidified with hydrochloric acid until pH 3, and then the mixture is cooled to a room temperature. Obtained residue is filtered, washed with water, and dried. Target product output was 11.5 g (30%).

The other claimed compounds are synthesized analogously, using other R₁; noticeable difference is observed only regarding output of the target product.

The compounds of the general formula (1) are colorless or slightly colored crystalline substances that are soluble in dimethyl-sulfoxide, chloroform, pyridine.

Individuality of the compounds is proved by thin-layer chromatography on the plates Silufol UV-254; the elutriator was carbon tetrachloride - isopropanol in the ratio 9:1.

The structure of the synthesized compounds is proved by the PMR spectroscopic method (see Table 2).

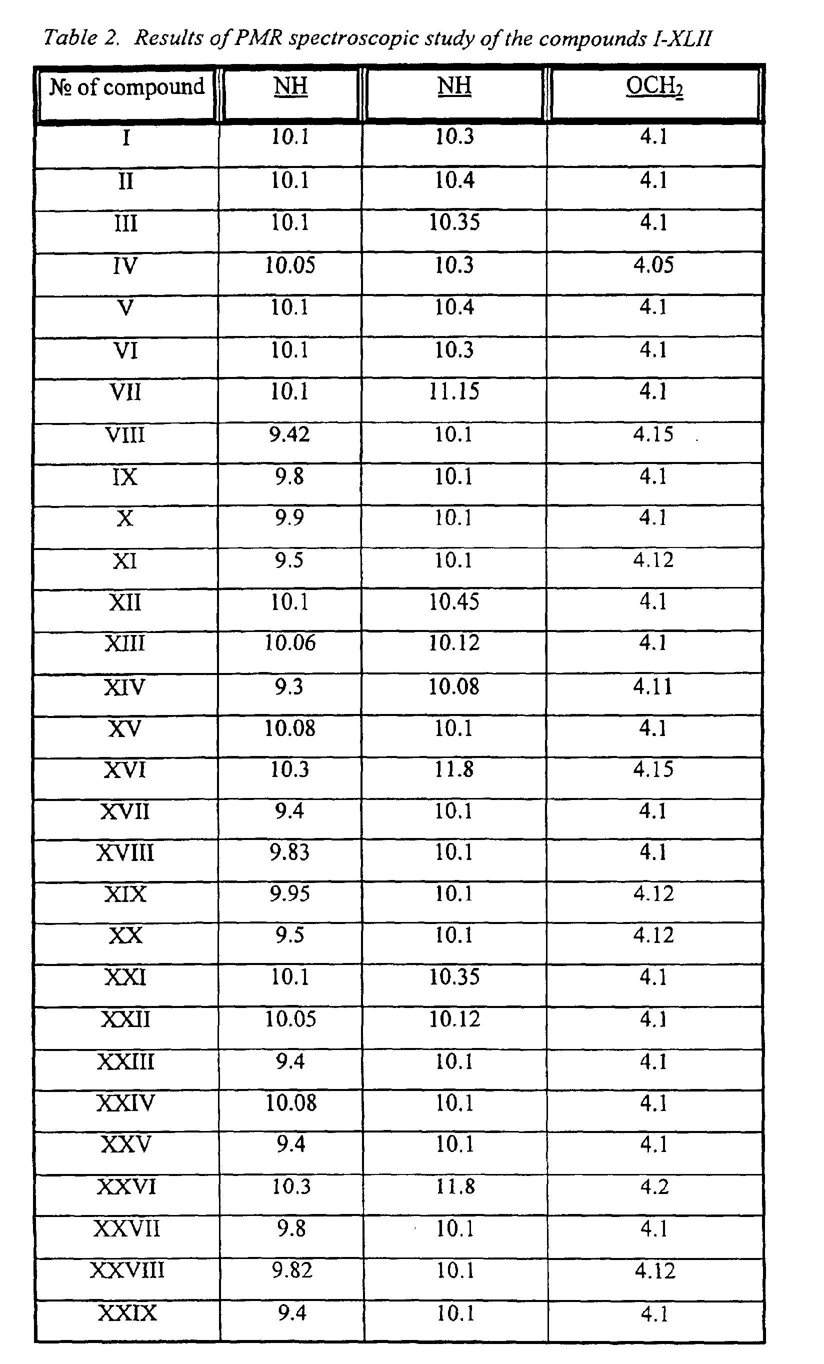

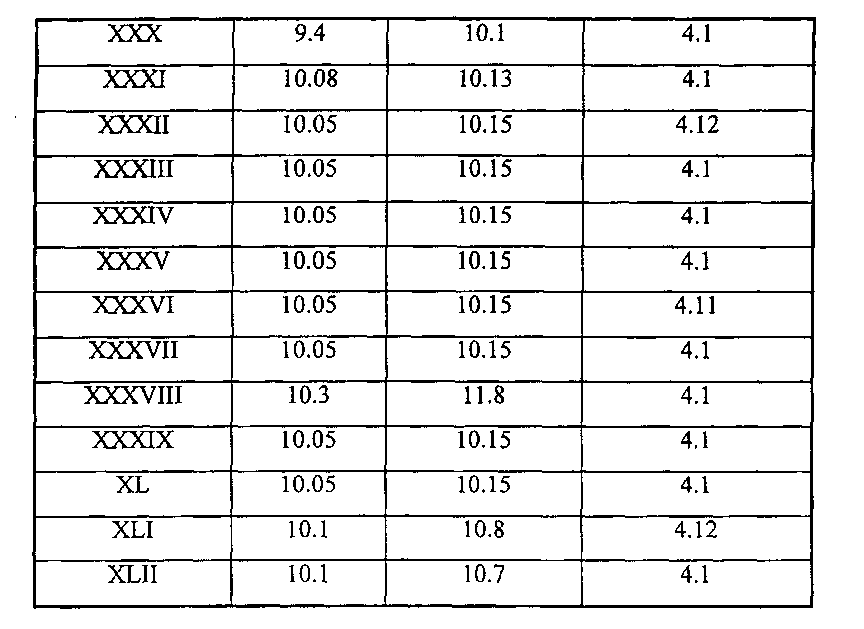

### Data on experimental determination of biological activity of the claimed compounds

### Experiment 1. Determination of antiviral effect of the claimed compounds, illustrated by the example of Herpes Simplex virus

An antiviral activity was determined with respect to *Herpes Simplex virus* of the type I (HSV-I /Leningrad/248/88) using commonly accepted method [7]. Viruses were grown on a continuous culture of Vero cells that have been received from the Bank of Cell Cultures of the Institute of Cytology of the Russian Academy of Science.

### Procedure of an experiment.

A virus in a final concentration of 10² TID₅₀/ml (TID is a tissue infection dose) and the claimed compounds, dissolved in DMSO in final concentrations of 100, 10 and 1 mg/l, were added to cells that were grown on the RPMI-1640 medium containing 10% of fetal calf serum and placed into wells of the 96-well plate. 5 separate wells were used for each of tested concentrations of an agent. The plate was incubated for 60 minutes at 37°C in a CO₂-incubator. After the incubation, the virus was removed and then a fresh medium containing claimed compounds in used concentrations was again introduced. Results were evaluated basing on presence of cytopathogenic action on the cells after 36 hours of incubation at 37°C in the CO₂-incubator.

The next controls were used in the experiment:
1. Control of cell culture (capability to grow properly)
2. Control of the virus (assessment of capability for reproduction)
3. Control of an antiviral activity of an antiviral drug Acyclovir
4. Control of compounds (toxicity of compounds)
5. Control of a solvent (DMSO) as to toxicity.

To estimate a cytopathic action of the virus, a quantity of the unchanged cells was calculated in 100 visual fields formed by a special net of an eyepiece-micrometer of an inverted microscope. The data obtained are shown in Table 3.

**Table 3.**

| *Effect of the claimed compounds on Herpes Simplex virus* | |
|---|---|
| Compound | Quantity of the unchanged cells (percentage of protection) at the compound concentration of 100 µg/ml |
| Cell control | 10000* |
| Acyclovir (10 µg/ml) | 8000* (80%) |
| DMSO | 10000 |
| III | 5000 (50%) |
| V | 5000 (50%) |
| VII | 8000 (80%) |
| VIII | 8000 (80%) |
| IX | 7000 (70%) |
| X | 7000 (70%) |
| XII | 5000 (50%) |
| XIII | 5000 (50%) |
| XIV | 7000 (70%) |
| XIX | 8000 (80%) |
| XXIX | 6000 (60%) |
| XL | 7000 (70%) |
| XLI | 7500 (75%) |

| | |
|---|---|
| * - number of cells in 100 visual fields under consideration ** - the percentage of protection of cells from virus as compared to the control of cell culture is shown in brackets. | |

The obtained results show that the claimed compounds presented in Table 3 possess antiherpetic activity which is comparable with that of a standard drug Acyclovir. The remainder of the claimed compounds demonstrated the less marked activity in suppressing reproduction of *Herpes virus* in the adopted experimental conditions.

### Experiment 2. Determination of interferon-inducing activity of the claimed compounds

Interferon synthesis induction caused by the claimed compounds was conducted on a primary culture of human lymphocytes (it is these cells in a human organism that are the main producers of interferons). To obtain lymphocyte culture, fresh blood (within 12 hours after sampling) from healthy donors (not of group II) was used. To separate lymphocytes, heparinized blood obtained from healthy donor was centrifuged in a density gradient ficoll-verografin of 1.71 g/cm³ in order to extract a fraction of immunocompetent cells. This fraction was selected and diluted with the nutrient medium RPMI-1640, containing 5% of fetal calf serum, 0.3 mg/ml of L-glutamin, 100 un/ml of penicillin, and 50 µg/ml of streptomycin. The concentration of lymphocytes was considered after dying them with methylene blue and calculation of cell number in a chamber of a hemocytometer. Initial solutions of the claimed compounds were diluted with the nutrient medium RPMI-1640 so that the final concentrations of the compounds, which were obtained after introduction of lymphocyte suspension, constituted the raw: 100 µg/ml, 10 µg/ml, 1 µg/ml. The final concentration of lymphocytes in the induction mixture was 3x10⁶ cell/ml.

In parallels to the test samples the following controls were done:
1) Control of spontaneous production of interferons (IFN) by lymphocytes.
2) Control of the process course under the action of the standardized IFN inducer N-methyl-N-(α,D-glucopyranosyl)ammonium-10-methylenecarboxylate acridone (Cyclopherone).
3) Control of the process course under the action of the standardized IFN inducer Neovir (sodium 10-methylenecarboxylate-9-acridone) with corresponding content of DMSO in experimental samples.
4) Control of spontaneous production of interferons in presence of DMSO taken in an amount corresponding to the test samples.

Control and test samples were incubated for 24 hours at the temperature of 37°C. After incubation, the samples were centrifuged at 2,000 G to deposit cell elements, and IFN-containing supernatant was separate out of the samples; then the supernatant was analyzed for the quantitative content of IFN. Cell deposition was resuspended in the preceding volume of nutrient medium, colored with vital dye trypan blue, and cell number was counted in a chamber of a hemocytometer (as described above) to determine cytotoxic action of the compounds.

Quantitative determination of the content of IFN in control and test samples was conducted using the immunoenzyme test system Pro Con IF2 Plus, that is intended for determination of IFN-α, which is produced by TOO "Protein contour". The solid-phase immunoenzyme method, utilizing horse radish peroxidase as an indicator enzyme, was used to determine the quantity of interferon in the samples. Activity of the bonded peroxidase was measured using an automatic photometer for microplates with a microprocessor at the wavelength of 450 nm.

To calculate the results, the activity of IFN in standard IFN solutions containing known quantities of preparation was determined in parallel. Based on the obtained results, a calibrating curve was formed, which allows to obtain data expressed in international units (IU) of activity, using the microprocessor of the automatic photometer. The results of the analysis are expressed in IU of activity of IFN per ml in the given induction system, which contains 3x10⁶ lymphocyte/ml. Each test and control point was investigated in four parallels.

Controls of immunoenzyme reaction:
1. Control of DMSO with a nutrient medium.
2. Control of system components (according to an instruction). All results were considered only if controls complied with passport data of the system.

The obtained data were undergone a statistical analysis using t-criterion, and calculation of the confident interval of probability at p=0.05 was carried out. Coincidence between the results obtained in the parallel tests was analyzed.

The investigations performed resulted in the fact, that, among the claimed substances, there are samples possessing capability to induce synthesis of IFN (Table 4).

**Table 4**

| *Quantitative evaluation of IFN-inducing activity of the claimed compounds* | | | |
|---|---|---|---|
| | Content of IFN in induction mixture after 24-hour incubation at various concentrations (µg/ml) of substances, IU/3x10⁴ lymph./ml | | |
| N Substance | 100 mg/l | 10 mg/l | 1 mg/l |
| Control of lymphocytes | 0 | 0 | 0 |
| Cycloferon | 70 ± 3 | 31 ±1 | 6,8 ± 0,6 |
| Neovir | 78 ± 1.6 | 36 ± 1,3 | 7,4 ± 0,8 |
| Poly I /poly C | -* | 48.6± 2.0 | 13.5 ± 1.4 |
| DMSO | 0 | 0 | 0 |
| III | 3,5 ± 0,2 | 2,6 ± 0,3 | 1,4 ± 0,2 |
| V | 2 ± 0,3 | 1,4 ± 0,1 | 1,1 ± 0,1 |
| VIII | 128 ± 2 | 31 ± 2 | 8 ± 0.6 |
| VIII | 1,7 ± 0,3 | 1,4 ± 0,2 | 1,5 ±0,2 |
| IX | 2,5 ± 0,5 | 2 ± 0,1 | 1,5 ± 0,1 |
| X | 100 ± 3 | 26 ± 1 | 7 ± 1 |
| XII | 2,5 ± 0,6 | 2 ± 0,5 | 1,3 ± 0,2 |
| XIII | 2 ± 0,1 | 1,4 ± 0,1 | 1,5 ± 0,2 |
| XIV | 2 ± 0,2 | 1,8 ± 0,2 | 1,2 ± 0,1 |
| XXVI | 2,5 ± 0,5 | 2 ± 0,2 | 1,6 ± 0,1 |
| XXVII | 2 ± 0,3 | 1,5 ± 0,3 | 1,2 ± 0,1 |
| XXVIII | 3 ± 0,1 | 2,3 ± 0,2 | 1,4 ± 0,1 |
| XL | 14 ± 0.2 | 3.6 ± 0.5 | 1.6 ± 0.1 |
| XLI | 69 ± 1 | 18.7 ± 0.2 | 5 ± 0.4 |

| | | | |
|---|---|---|---|
| *- compound of given concentration was not tested | | | |

The other claimed compounds have less marked interferon-inducing activity.

### Experiment 3. Determination of the action of the claimed compounds against chlamydia, illustrated by the example of Chlamydia trachomatis.

The antichlamydial activity of the claimed compounds was studied in respect to C.trachomatis D323, which is a standard strain contained in the collection of the department of microbiology of St. Petersburg State Pavlov Medical University. This strain was derived >from a patient having Chlamydia urethritis; the strain has a morphology and physiological activity that are characteristic for representatives of this type; the strain is sensitive to action of compounds that are used to treat chlamydia infection. Cell cultures McCoy and L929 obtained from the Institute of Cytology of RAS were used in this work.

### Procedure of an experiment.

Cells were grown in flasks made of neutral glass in the medium RPMI-1640 containing 10% of fetal calf serum. The test was performed in flat-bottom flasks made of glass (non-toxic), that were supplied with cover glasses. The cells were introduced into the medium in the final concentration of 1x10⁶ cell/ml. After a monolayer had been obtained, standard infectious dosages of chlamydia, which had been stored in a frozen state at the temperature of -70°C, were introduced into test tubes. Simultaneously, tested compounds in a final concentration of 100 mg/l were added to the cells. The sample was centrifuged at 2400 G for 60 minutes at a room temperature and incubated at 37°C for 2 hours. After this, the nutrient medium was replaced with a new medium, containing 5% of fetal calf serum and cycloheximide (2 µg/ml), with repeated introduction of the claimed compounds in the preceding concentration. The samples were doubled using the medium without cycloheximide so as to exclude its effect on the investigated compounds. The samples were incubated for 48 hours in CO₂-incubator.

The controls included:
1. Control of cell culture
2. Control of action of solvents
3. Control of action of chlamydia in absence of any compounds
4. Control of sensitivity of chlamydia to standard antimicrobial compound Ciprofloxacin
5. Control of the tested compounds as to toxicity in respect to the cell culture.

Evaluation of the results was carried out by the way of determination of chlamydial cytoplasmatic inclusions (CPI) that was conducted by the method of immunofluorescence (MicroTrack Chlamydia trachomatis Direct Specimen Test), and by the way of determination of chlamydia antigens that was conducted by means of CylaMonoScreen (Russian-British Joint Venture 66 Regent's Parc Road London NW1 7SX) [8, 9]. The effect of the action of compound was determined by analysis of the monolayer state and number of cells with CPI when compared with the control (cell culture infected with C.trachomatis D323); in doing so, the number of unchanged cells, that were counted in 100 visual fields obtained using the eyepiece-micrometer, was taken into consideration.

Results of the control samples satisfying to the requirements of the experiment, are the following:
- control of cell culture: morphology of cells and state of monolayer correspond to the given type of the cells;
- control of chlamydia growth in the cell culture: presence of CPI in the monolayer;
- control of action of standard antimicrobial agent: reduction of number of CPI in the monolayer as compared to the preceding control;
- control of toxicity of the claimed compounds: toxicity is absent;
- control of laction of solvents: toxic action on cells is absent.

The results of performed tests are presented in Table 5.

**Table 5.**

| *Effect of claimed substances on C.trachomatis* | |
|---|---|
| NN Substance | Number of unchanged cells |
| 1. Cell control | 8.000 (100%) |
| 2. DMSO | 8.000 (100%) |
| 3. Control of infected cells | 1.500 (18.75%)** |
| 4. Ciprofloxacin (100)* | 4.000 (50%)** |
| 5. X | 4.000 (50%)** |
| 6. XII | 3.600 (45%)** |
| 7. XXIX | 4.400 (55%)** |
| 8. XLI | 4.400 (55%)** |

| | |
|---|---|
| * - concentration of the preparation, mg/l | |
| ** - percentage of cell protection from infection is given in brackets. | |

The data obtained give an evidence of the fact that the claimed compounds XXIX and XLI are of marked biological activity against chlamydia, and this activity is superior to the activity of the standard drug Ciprofloxacin.

The other claimed compounds possess less marked activity as to protection of cells from chlamidia in the adopted conditions of experiment.

### Experiment 4. Determination of hepatoprotective activity of the claimed compounds

CCl₄ dissolved in 50% olive oil (1 ml/kg) was subcutaneously administrated to rats. Test substances were administrated perorally in a dose of 20 mg/kg 30 minutes before and 7 hours after the CCL₄ administration. The activity of alanine-aminotranspherase (AlAT), which is a marker enzyme of liver parenchyma damage, was assessed twenty-four hours later. Reducing of enzyme amount by more than 30% relative to the control group was considered as evidence of hepatoprotective properties of the substance. Vitamin E in a dose of 100 mg/kg was used as a reference agent. Experimental results are shown in Table 6.

**Table 6.**

| *Assessment of hepatoprotective activity of the claimed substances*. | |
|---|---|
| Group | % of reducing AlAT |
| Intact | - |
| CCl₄ | - |
| XXIX | -36 |
| Vitamin E | -30 |

The other claimed compounds possess less pronounced hepatoprotective activity.

### Experiment 5. Determination of antiaggregational properties of the claimed compounds

Human's platelet aggregation was investigated in plasma of healthy 50±3 years donors that was enriched by thrombocytes, by means of aggregometer FRM-1. The substance under investigation was added to blood plasma samples (100 µg/ml) before inducing aggregation by means of adenosine diphosphate (2.5 µmol/l). Magnitude of the maximum non-reversible platelet aggregation in the control samples (solvent) was adopted as 100%, and that in tested samples was counted as percentage compared to the control. Inhibition of the aggregation by more than 50% at the given concentration was evaluated as meaningful inhibition. Adenosine (5 µg/ml) and aspirin (9 µg/ml), that inhibit aggregation at these concentrations by 100%, were utilized as reference agents.

**Table 7.**

| *Antiaggregational action of the claimed substances.* | | |
|---|---|---|
| Substance | Concentration, µg/ml | % of inhibition |
| XXIX | 100 | 33.3±8.2 |
| Adenosine | 5 | 100 |
| Aspirin | 9 | 100 |

The other claimed compounds were not tested specifically for the antiaggregational activity, but we have some data that each of the claimed compounds possess antiaggregational action to a greater or lesser degree.

### Experiment 6. Determination of antimicrobial action of the compounds

To determine an antimicrobial activity a standard strain *Mycobacterium tuberculosis* H37RV, which is sensitive to all the antimicrobial agents, was used. Antimycobacterial action was estimated by the serial dilution method.

Substances were dissolved in dimethyl-sulfoxide (DMSO) and titrated in the medium N-1, so that the preparation was contained in separate test tubes with the medium in concentrations >from 200 to 0.025 mg/l. The concentrations of the substance in the medium in adjacent test tubes differed by the factor of two. DMSO titrated in the same way as the substance was utilized for the control. The result was considered after a 72-hour cultivation of the bacteria at 37°C.

*M.tuberculosis* H37Rv were grown on the Soton medium which contained 10% of horse serum, and the density of microbial suspension when seeding was 50x10⁶ cell/ml.

Tuberculostatic compounds that are known were utilized as the controls. The results obtained for the used strain are summarized in Table 8.

**Table 8.**

| *Minimal ingibitory concentration (MIC) regarding M.tuberculosis H37R (mg/l)* | | |
|---|---|---|
| | Compound | MIC, mg/l |
| | XXXIV | 50 |
| | Streptomycin | 0.2 |
| | Isoniazid | 0.1 |
| | Rifampycin | 0.05 |
| | Ethambutol | 5.0 |
| | Ofloxacin | 0.5 |

The data shown in Table 8 indicate that the compound XXXIV possesses antimicrobial activity in respect to the used strain of M. *tuberculosis* in concentrations of 50 mg/l.

No antimicrobial activity of the other claimed substances in respect to *M*.*tuberculosis* was revealed.

### Experiment 7. Determination of maximal tolerable dose

Tested substance was introduced perorally (in dose of 300 mg/kg) through a gastric tube or intraperitoneally (in dose of 100 mg/kg) to no-breed white mice with mass 18-20 g (each tested group consisted of 3 males and 3 females). Then the mouse state was observed during 72 hours. Absence of symptoms characteristic of toxic effects, or absence of animal death during the given time allows to make a conclusion that the toxicity of the tested substance is low [10].

**Table 9.**

| *Maximal tolerable dose* | |
|---|---|
| Compound | Concentration of tested compounds (mg/l) at oral introducing |
| Tween 80 | 300* |
| VII | 300 |
| VII | 300 |
| X | 300 |
| XIX | 300 |
| XXI | 300 |
| XXII | 300 |
| XXVI | 300 |
| XXVIII | 300 |
| XXXIII | 300 |
| XL | 300 |
| XLI | 300 |

| | |
|---|---|
| * In all the cases including control the concentration of 300 mg/l was the maximally utilized concentration. | |

The other claimed compounds demonstrated similar toxicity.

The obtained results show that the claimed compounds given by oral administration in the dose of 300 mg/l do not possess an acute toxicity on mice.

### INDUSTRIAL APPLICABILITY

The examples given above and practical results of synthesis and analysis of the claimed substances confirm possibility of laboratory and industrial synthesis of the claimed substances by the means that have been leant by modern pharmaceutical industry, and also their clear identification by commonly used control methods.

Series of experiments on determination of biological activity have shown, that the claimed compounds possess biological activity against various microorganisms, that are *Herpes Simplex virus*, mycobacteria, and chlamydia, marked interferon-inducing and hepatoprotective activities, and also antiaggregational properties. High interferon-inducing activity of these compounds indicates also the possibility of their using for treatment some of oncologic diseases. Toxicity and maximal tolerable dose have been determined.

The presented data prove that the objectives stated by the invention have been achieved: new chemical compounds, which are aryl- and heterylamides of carboalkoxysulfanilic acids, were synthesized, and these compounds possess low toxicity and a wide range of pronounced biological action, particularly antiviral, immune stimulating, antibacterial, antichlamydial, antiaggregational, hepatoprotective, and antimicrobial activities.

Therefore, the claimed substances satisfy all the demands that are imposed upon an invention: they are new, not obvious, and industrially applicable.

### Literature

1□ Chatis P.A., Crumpacker C.S. Resistance of herpesviruses to antiviral drugs. Antimicrob. Agents Chemother. 1992; 36: 1589-1595.
2□ Pharmaceutical microbiology. Ed. by W.B.Hugo and A.D.Russel Blackwell Scientific Publications, Oxford, 1987, 511 p.
3□ Esteban M., Paez E. Antiviral and antiproliferartive properties of interferons: mechanism of action. Prog. med. virol. 1985, 32:159-173.
4□ Tanneberg S., Hrelia P. Interferons in precancer and cancer prevention: where are we? J.of Interferon and Cytokine Res. 1996, 16,5:339-346.
5□ Mashkovsky M.D., "Drug Means." (in 2 parts, in Russian). Moscow, "Medicine", 1993, part 1, p.520.
6□ Vaughan J.R.,Eichen J.A. and Anderson G.W., Heterocyclic Sulfonamides as Carbonic Anhydrase Inhibitors 2-Acylamido-and 2-Sulfonamodo-1,3,4-thiadiazole-5-Sulfonamides, The Journal of Organic Chemistry,1956.V.21. P.700 - **PROTOTYPE.**
7□ Gentry G.A., Lawrency N., Lushbaugh N. Isolation and differentiation of Herpes simplex virus and Trichomonas vaginalis in cell culture, J. of Clinical Microbiology 1985, Vol. 22, No. 2, P. 199-204.
8□ Wang S-P., Grayston J.T. Serotyping of Clamydia trachomatis by indirect fluorescent-antibody staining of inclusions in cell culture with monoclonal antibodies. J. of Clinical Microbiology, 1991, Vol.29, No. 7, P.1295-1298.
9□ Judson B.A., Lambert P.P. Improved Syva MicroTrac Clamydia trachomatis direct test method, Journal of Clinical Microbiology, 1988, Vol.26, No. 12, P.2657-2658 10□Irwin S. Psychopharmacology, 1968, 13, P. 222-257.

## Claims

1. Aryl- and heterylamides of carboalkoxysulfanilic acids of the general formula (1) 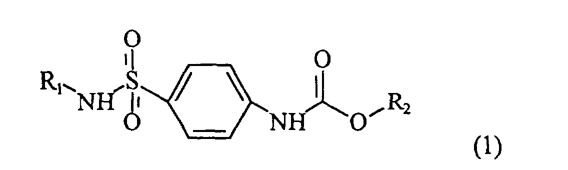 where:
R₁ is taken from the group of aryls or heteryls;
R₂ is taken from the group of alkyls.

2. □ Compound of claim 1 differing in R₁ = 4-BrPh, R₂ = CH₃

3. □ Compound of claim 1 differing in R₁ = 4-BrPh, R₂ = C₂H₅

4. □ Compound of claim 1 differing in R₁ = 4-BrPh, R₂ = C₄H₉

5. □ Compound of claim 1 differing in R₁ = 4-BrPh, R₂ = C₅H₁₁

6. □ Compound of claim 1 differing in R₁ = 4-BrPh, R₂ = C₆H₁₃

7. □ Compound of claim 1 differing in R₁ = 4-BrPh, R₂ = C₇H₁₅

8. □ Compound of claim 1 differing in R₁ =4-NO₂Ph, R₂ = C₄H₉

9. □ Compound of claim 1 differing in R₁ =2-CH₃Ph, R₂ = C₄H₉

10. □ Compound of claim 1 differing in R₁ =2-ClPh, R₂ = C₄H₉

11. □ Compound of claim 1 differing in R₁ =2,6-Cl₂Ph, R₂ = C₄H₉

12. □ Compound of claim 1 differing in R₁ =2-IPh, R₂ = C₄H₉

13. □ Compound of claim 1 differing in R₁ = 3-BrPh, R₂ = C₄H₉

14. □ Compound of claim 1 differing in R₁ =Ph, R₂ = C₄H₉

15. □ Compound of claim 1 differing in R₁ =2,5-(CH₃)₂Ph, R₂ = C₄H₉

16. □ Compound of claim 1 differing in R₁ =4-FPh, R₂ = C₄H₉

17. □ Compound of claim 1 differing in R₁ =1,2,4-Triazole-4-yl, R₂ = C₅H₁₁

18. □ Compound of claim 1 differing in R₁ =2-CH₃Ph, R₂ = C₅H₁₁

19. □ Compound of claim 1 differing in R₁ =2-ClPh, R₂ = C₅H₁₁

20. □ Compound of claim 1 differing in R₁ =2,6-Cl₂Ph, R₂ = C₅H₁₁

21. □ Compound of claim 1 differing in R₁ =2-IPh, R₂ = C₅H₁₁

22. □ Compound of claim 1 differing in R₁ =3-BrPh, R₂ = C₅H₁₁

23. □ Compound of claim 1 differing in R₁ =Ph, R₂ = C₅H₁₁

24. □ Compound of claim 1 differing in R₁ =2,5-(CH₃)₂Ph, R₂ = C₅H₁₁

25. □ Compound of claim 1 differing in R₁ = 4-FPh, R₂ = C₅H₁₁

26. □ Compound of claim 1 differing in R₁ =2-CH₃Ph, R₂ = CH₃

27. □ Compound of claim 1 differing in R₁ =1,2,4-Triazole-4-yl, R₂ = C₂H₅

28. □ Compound of claim 1 differing in R₁ =2-ClPh, R₂ = C₇H₁₅

29. □ Compound of claim 1 differing in R₁ =2-ClPh, R₂ = C₆H₁₃

30. □ Compound of claim 1 differing in R₁ =2-CH₃Ph, R₂ = C₆H₁₃

31. □ Compound of claim 1 differing in R₁ =2,5-(CH₃)₂Ph, R₂ = C₆H₁₃

32. □ Compound of claim 1 differing in R₁ =Ph, R₂ = C₆H₁₃

33. □ Compound of claim 1 differing in R₁ =1,2,4-Thiadiazole-2-methyl-5-yl, R₂ = C₄H₉

34. □ Compound of claim 1 differing in R₁ =1,2,4-Thiadiazole-2-butyl-5-yl, R₂ = CH₃

35. □ Compound of claim 1 differing in R₁ =1,2,4-Thiadiazole-2-hexyl-5-yl, R₂ = C₇H₁₅

36. □ Compound of claim 1 differing in R₁ =1,2,4-Thiadiazole-2-pentyl-5-yl, R₂ = isoC₄H₉

37. □ Compound of claim 1 differing in R₁ =1,2,4-Thiadiazole-2-methyl-5-yl, R₂ = CH₃

38. □ Compound of claim 1 differing in R₁ =1,2,4-Thiadiazole-2-butyl-5-yl, R₂ = C₆H₁₃

39. □ Compound of claim 1 differing in R₁ =1,2,4-Triazole-4-yl, R₂ = C₄H₉

40. □ Compound of claim 1 differing in R₁ =1,2,4-Thiadiazole-2-pentyl-5-yl, R₂ = CH₂CF₃

41. □ Compound of claim 1 differing in R₁ =1,2,4-Thiadiazole-2-butyl-5-yl, R₂ = CH₂CF₃

42. □ Compound of claim 1 differing in R₁ =1,5-Dimethyl-2-phenylpyrazolone-4-yl, R₂ = C₅H₁₁

43. □ Compound of claim 1 differing in R₁ =1,3,5-Triazine-2,4-diamino-6-yl, R₂ = C₅H₁₁
